# EUROPEAN PATENT SPECIFICATION

(11) **EP 3 081 616 B1**
(45) Date of publication and mention of the grant of the patent: **06.06.2018**
(21) Application number: 15382188.9
(22) Date of filing: 17.04.2015
(51) Int. Cl.: C09K 11/06, H01L 51/00, A61K 49/00, H01L 51/50

(54) **WHITE LIGHT EMITTING ZIRCONIUM-BASED MOFS**
AUF ZIRCONIUM BASIERENDE WEISSLICHTEMITTIERENDE MOFS
MOFS D'ECLAIRAGE A LUMIERE BLANCHE A BASE DE ZIRCONIUM

(43) Date of publication of application: 19.10.2016
(73) Proprietor: Universidad de Castilla La Mancha, 02071 Albacete (ES)
(72) Inventor: Douhal Alaui, Abderrazzak, 02071 Albacete (ES); Gutiérrez Tovar, Mario, 02071 Albacete (ES)
(74) Representative: Balder IP Law, S.L.

(56) References cited:
- US-A1- 2010 226 991
- XIU-LIANG LV ET AL: "A high surface area Zr(IV)-based metal-organic framework showing stepwise gas adsorption and selective dye uptake", JOURNAL OF SOLID STATE CHEMISTRY, vol. 223, 1 March 2015 (2015-03-01), pages 104-108, XP055215064, ISSN: 0022-4596, DOI: 10.1016/j.jssc.2014.07.001
- JIAO HE ET AL: "A dye-sensitized Pt@UiO-66(Zr) metal-organic framework for visible-light photocatalytic hydrogen production", CHEMICAL COMMUNICATIONS, vol. 50, no. 53, 1 January 2014 (2014-01-01), pages 7063-7066, XP055215065, ISSN: 1359-7345, DOI: 10.1039/c4cc01086h

## Description

### FIELD OF THE INVENTION

The present invention relates to composite materials emitting white light based on metal-organic frameworks (MOF) of Zr, to a method for their preparation and to applications thereof, such as in sensors or imaging.

### BACKGROUND

The use of light emitting materials in different technological systems such as fluorescent sensors or light emitting devices have aroused great interest for the last two decades. Providing stable, high efficiency and low power consumption light emitting materials is one of the aims of many research groups. A promising alternative to current systems are the metal-organic frameworks (MOFs), which open new possibilities to further improve the properties of commercial light emitting devices.

MOFs have attracted considerable attention owing to their excellent structural properties, such as high specific surface areas, large pore volumes as well as their free and accessible cavities (Zhou, H. C.; Long, J. R.; Yaghi, O. M., Introduction to metal-organic frameworks. Chem. Rev. (washington, DC, U. S.) 2012, 112 (2), 673-674). These properties make them promising materials for a wide range of applications, for example, as chemical sensors and optoelectronic devices. Thus, for the past two decades different MOFs containing specific ligands/metals and having different pore sizes and internal cavities have been developed (Cook, T. R.; Zheng, Y.-R.; Stang, P. J., Metal-Organic Frameworks and Self-Assembled Supramolecular Coordination Complexes: Comparing and Contrasting the Design, Synthesis, and Functionality of Metal-Organic Materials. Chem. Rev. (Washington, DC, U. S.) 2013, 113 (1), 734-777; and Furukawa, H.; Cordova, K. E.; O'Keeffe, M.; Yaghi, O. M., The Chemistry and Applications of Metal-Organic Frameworks. Science 2013, 341 (6149)).

Also, MOFs can be used as the luminescent layer for light emitting diode (LED) devices. Thus, improving both their fabrication and luminescent properties has been the object of numerous investigations. The main advantage of using MOF complexes in the White-LED manufacturing is the need of only one composite material instead of three phosphorescent (blue, green and red) currently needed (Kim, B. S.; Lee, J. Y., Interlayer free hybrid white organic light-emitting diodes with red/blue phosphorescent emitters and a green thermally activated delayed fluorescent emitter. Organic Electronics 2015, 21 (0), 100-105).

For example, in US2010/226991, MOF molecules which can encapsulate fluorescent molecules such as rhodamine perchlorate or fluorescein. The emission spectra of the complexes of MOF MIL-101-NH2 with rhodamine 116, fluorescein, PSO3 or APNAF are studied, but none of them is capable of providing white light. Neither it is suggested that such light can be obtained. Xiu-Lian Lv et al, Journal of solid state chemistry, 2015, 223, 104-108 discloses MOFs used in purification processes. No suggestion is given in relation to the possibility of obtaining MOFs which can provide white light emissions. Jiao He et al, Chem. Commun. 2014, 50(53), 7063-7066 discloses UiO-66@rhodamine B complexes having photocatalytic properties, but nothing is suggested about white light emission.

Owing to their microporous structure, MOFs can act as a host for luminescent dyes opening the possibility to tune the photoluminescent properties of both. For example, the encapsulation of an Ir-complex into the porous matrix of a Cd-based MOF supplies a white light emissive material (Sun, C. Y.; Wang, X. L.; Zhang, X.; Qin, C.; Li, P.; Su, Z. M.; Zhu, D. X.; Shan, G. G.; Shao, K. Z.; Wu, H.; Li, J., Efficient and tunable white-light emission of metal-organic frameworks by iridium-complex encapsulation. Nature Communications 2013, 4). CN 103 740 361 discloses a In-based MOF in which pyridine hemicyanine and acridine dyes are incorporated into the related MOF porous structure, providing a white light emissive material. Methods for the synthesis of such materials can be long and complex.

Further, MOF's ability to sense small molecules, volatile organic compound (VOCS) and explosives (TNT) has been proven. Zr-based MOF have been used to sense the presence of small molecules (Zhang, W.; Huang, H.; Liu, D.; Yang, Q.; Xiao, Y.; Ma, Q.; Zhong, C., A new metal-organic framework with high stability based on zirconium for sensing small molecules. Microporous Mesoporous Mater. 2013, 171 (0), 118-124). The materials described therein did not incorporate dyes difficult to manufacture. The sensitivity towards explosive derivatives (e.g., TNT) has been studied in Zn- or Cd-based MOFs wherein the MOF photoluminescence is highly quenched by the presence of these explosive molecules (Gole, B.; Bar, A. K.; Mukherjee, P. S., Fluorescent metal-organic framework for selective sensing of nitroaromatic explosives. Chem. Commun. (Cambridge, U. K.) 2011, 47 (44), 12137-12139.; Nagarkar, S. S.; Joarder, B.; Chaudhari, A. K.; Mukherjee, S.; Ghosh, S. K., Highly Selective Detection of Nitro Explosives by a Luminescent Metal-Organic Framework. Angewandte Chemie International Edition 2013, 52 (10), 2881-2885; and Lan, A.; Li, K.; Wu, H.; Olson, D. H.; Emge, T. J.; Ki, W.; Hong, M.; Li, J., A Luminescent Microporous Metal-Organic Framework for the Fast and Reversible Detection of High Explosives. Angewandte Chemie International Edition 2009, 48 (13), 2334-2338). Moreover, MOFs are also useful in imaging, and different Gd-based MOFs have been tested (Rieter, W. J.; Taylor, K. M. L.; An, H.; Lin, W.; Lin, W., Nanoscale Metal-Organic Frameworks as Potential Multimodal Contrast Enhancing Agents. J. Am. Chem. Soc. 2006, 128 (28), 9024-9025; T aylor, K. M. L.; Jin, A.; Lin, W., Surfactant-Assisted Synthesis of Nanoscale Gadolinium Metal-Organic Frameworks for Potential Multimodal Imaging. Angewandte Chemie International Edition 2008, 47 (40), 7722-7725).

Therefore, alternative and improved MOF-based materials are needed which further improve those currently known and provide less complex manufacturing methods.

### SUMMARY OF THE INVENTION

In order to overcome the above mentioned problems, the inventors have developed an alternative MOF-based composite material to improve the current situation. The invention relates to a Zr-based metal-organic framework (MOF) in whose porous structure dyes, preferably organic laser dyes, are trapped. The composite materials of the invention display a high quality white light emission. According to a first aspect the invention provides a white light emitting device comprising a composite material emitting white light comprising a MOF comprising a plurality of organic polydentate bridging ligands and a plurality of zirconium cations, characterized in that said composite material comprises at least one guest dye capable of absorbing energy in a wavelength range emitted by the MOF when said MOF is excited, said at least one guest dye comprising a chromophore selected from the group consisting of a naphthoxazinium group, an aromatic cyclobutadione group, a 1-benzopyran-2-one group, xanthenylidene-dialkylammonium group, a porphyrin group, a 4-dimethylaminostyryl group or boron-dipyrromethene group (also known as BODIPYs) and mixtures thereof.

The composite materials of the invention are easy to make and provide the composite material in good yield and short time, and high adsorption rate of the guest dyes (or dyes). It is thus a second aspect of the invention to provide a method for preparing the composite material, comprising contacting said MOF comprising a plurality of organic polydentate bridging ligands and zirconium, with at least one guest dye as defined in the first aspect.

The composite materials of the invention provide high quality white light and can thus be used for manufacturing a white light emitting device, which is a third aspect of the invention. A fourth and fifth aspects of the invention are the use of the white light emitting device comprising the composite material for manufacturing a sensor, and such sensor, respectively

### DETAILED DESCRIPTION OF THE INVENTION

### Definitions

"Alkyl" is given its usual meaning according to the IUPAC Gold Book and refers to a straight or branched univalent groups derived from alkanes by removal of a hydrogen atom from any carbon atom having the formula -CₙH₂ₙ₊₁. Exemplary alkyl groups can be methyl, ethyl, n-propyl, i-propyl, n-butyl, t-butyl, n-pentyl, etc. The alkyl group can be optionally perfluorated.

"Alkenyl" refers to a straight or branched hydrocarbon chain radical consisting of carbon and hydrogen atoms, containing at least one unsaturation (e.g. one, two, three or more), and which is attached to the rest of the molecule by a single bond. Exemplary alkenyl groups are vinyl, neoprenyl, 2-propenyl, 1,3-butadiene or isopropenyl.

"Alkynyl" refers to a straight or branched hydrocarbon chain radical consisting of carbon and hydrogen atoms, containing at least one triple bond (e.g. one, two, three or more), and which is attached to the rest of the molecule by a single bond. Exemplary alkenyl groups are acyl or 2-propynyl.

"Aryl group" refers to an aromatic hydrocarbon radical such as phenyl or naphthyl.

"Heteroaryl group" refers to a stable 3- to 15- membered ring, wherein at least one of said rings is aromatic, which consists of carbon atoms and from one to five heteroatoms selected from the group consisting of nitrogen, oxygen, and sulphur, preferably a 4-to 8-membered ring with one or more heteroatoms, more preferably a 5-or 6-membered ring with one or more heteroatoms. For the purposes of this invention, the heterocycle may be a monocyclic, bicyclic or tricyclic ring system, which may include fused ring systems; and the nitrogen, carbon or sulfur atoms may be optionally oxidised; the nitrogen atom may be optionally quaternized; and the rings may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran, furan or 2-Benzothiazolyl.

"Alkylaryl" or "arylakyl" group refers to an aryl group linked to an alkyl group, the alkylaryl group being attach to the rest of the molecule through either of the alkyl or the aryl moiety. Examples are benzyl or phenethyl. Also groups alternating different alkyl and aryl moieties, including cycloalkyl groups, such as 3-[3-[4-(4-Bromophenyl)phenyl]-1,2,3,4-tetrahydronaphthalen-1-yl]- or 3-[3-[4-(4-Bromophenyl)phenyl]-3-hydroxy-1-phenylpropyl]-.

"Alkoxy" refers to a radical of the formula -ORa where Ra is an alkyl, alkenyl, alkynyl, aryl or arylalkyl having one to twelve carbon atoms, e. g., methoxy, ethoxy, propoxy, benzyloxy etc.

"Halogen" takes its usual meaning and refers to any of the atoms of group 17 selected form -F, -Cl, -Br and -I.

"Heterocyclyl" refers to a heteroaryl group having no aromatic rings.

"Cycloalkyl" is given its usual meaning according to the IUPAC Gold Book and refers to a univalent group derived from cycloalkanes by removal of a hydrogen atom from a ring carbon atom. Examples of cycloalkyl groups are cyclopropyl, cyclopentyl or cyclo hexyl.

Where indicated, the size of the above groups is indicated by the minimum and maximum number of carbons comprised in each is case. This is indicated in the usual way by noting the group has Cₙ-Cₘ carbon atoms, wherein n is the minimum number of carbon atoms, and m is the maximum number of carbon atoms.

### Composite material

The composite materials of the invention comprises thus two main parts, the MOF structure which comprises a plurality of organic polydentate bridging ligands and a plurality of zirconium cations, and the guest dyes (or dyes).

MOFs are porous materials built from metal ions with a defined coordination geometry, in the present invention a zirconium cations, and organic polydentate bridging ligands. The coordination between the metal ion and the ligands extend in one, two or three dimensions creating a network whose properties can be tuned. Many publications provide general descriptions of MOF materials. Examples are Zhou, H. C.; Long, J. R.; Yaghi, O. M., Introduction to metal-organic frameworks. Chem. Rev. (Washington, DC, U.S.) 2012, 112 (2), 673-674); Cook, T. R.; Zheng, Y.-R.; Stang, P. J., Metal-Organic Frameworks and Self-Assembled Supramolecular Coordination Complexes: Comparing and Contrasting the Design, Synthesis, and Functionality of Metal-Organic Materials. Chem. Rev. (washington, DC, U. S.) 2013, 113 (1), 734-777; or Furukawa, H.; Cordova, K. E.; O'Keeffe, M.; Yaghi, O. M., The Chemistry and Applications of Metal-Organic Frameworks. Science 2013, 341 (6149)).

For the purposes of the present invention any Zr-based MOF is suitable, and the skilled person can choose a wide array of organic polydentate bridging ligands. Those preferred in the present invention allow in combination with the Zirconium cations the formation of a three dimensional network or two dimensional which can host dyes (e.g. nanotubes). Examples of organic polydentate bridging ligand families are according to an embodiment of the invention bidentate carboxylic acids, tridentate carboxylic acids or azoles. Bidentate carboxylic acids can be divided in non-aromatic and aromatic, of which ethanedioic acid, propanedioic acid, butanedioic acid or pentanedioic acid are examples of the former, and benzene-1,2-dicarboxylic acid-*o*-phthalic acid, benzene-1,3-dicarboxylic acid-*m*-phthalic acid, benzene-1,4-dicarboxylic acid-*p*-phthalic acid, 2,6-naphthalene dicarboxylic acid, 1,4-benzene dicarboxylic acid, biphenyl-4,4-dicarboxylic acid or 1-amine-2,6-naphthalene dicarboxylic acid are examples of the latter. Tridentate carboxylic acids can also be divided in non-aromatic (e.g. 2-Hydroxy-1,2,3-propanetricarboxylic acid) and aromatic (e.g. benzene-1,3,5-tricarboxylic acid). All the above make embodiments of the present invention. According to further embodiments of the invention, the organic polydentate bridging ligands are a dicarboxylic aromatic compounds, preferably one selected from the group consisting of 2,6-naphthalene dicarboxylic acid, 1,4-benzene dicarboxylic acid, Biphenyl-4,4-dicarboxylic acid, 1-amine-2,6-naphthalene dicarboxylic acid, and mixtures thereof, more preferably one comprising a napthane in its structure. According to a preferred embodiment, at least one of said plurality of organic polydentate bridging ligands is 2,6-naphthalene dicarboxylic acid (NDC).

The composite material of the invention adsorbs in the pores of the MOF at least one guest dye capable of absorbing energy of the same or similar wavelength as emitted by the MOF when said MOF is excited and of emitting light in a wavelength which, in combination with the light emitted by the MOF and the other guest dyes, creates white light. The inventors have discovered that Zr-based MOFs provide an appropriate template for hosting dyes, and the election of guest dyes emitting in the appropriate wavelength provides in combination with the MOF an excellent white light. For example, Zr-NDC emits when excited with a UV-visible source around 335 nm, i.e. in the blue zone of the visible spectrum. The election of a guest dye which can absorb the energy emitted by the MOF upon excitation at 335 nm and emit at a wavelength in the red zone of the spectrum (e.g. 600nm-800nm) and the green zone (e.g. 460-600 nm) can create with a single material white light. Such emissions can come from a single dye or from different dyes. Dyes are marketed with information about their excitation and emission wavelengths, which is displayed in internet or provided as brochures. See for example, http:// docs.abcam.com/ pdf/immunology /fluorochromes table updated.pdf. Thus, the skilled person can choose the dye having the appropriate absorption and emission wavelengths which in combination with the Zr-MOF will provide white light.

The composite material of the invention emits excellent quality white light. Usually, the energy absorption by the dye from the MOF takes place in at least one single step from the MOF to one of the dyes, takes place in cascade from the MOF to a first dye and from said first dye to a second dye, or wherein said energy absorption takes place in a combination of at least one single steps and at least one cascade. According to an embodiment of the invention, the MOF emits in a wavelength comprised between 350 and 550 nm. In a further embodiment, the composite material comprising two or more guest dyes wherein at least one of the guest dyes absorbs energy in a wavelength comprised between 350 and 550 nm and emits in a wavelength comprised between 460 and 650 nm, or two or more guest dyes wherein at least one of the guest dyes absorbs energy in a wavelength comprised between 350 and 550 nm and emits in a wavelength comprised between 600 and 800 nm. In a preferred embodiment, the composite material comprises two or more guest dyes wherein at least one of the guest dyes absorbs energy in a wavelength comprised between 350 and 550 nm and emits in a wavelength comprised between 600 and 800 nm and wherein at least one of the guest dyes absorbs energy in a wavelength comprised between 350 and 550 nm and emits in a wavelength comprised between 460 and 600 nm.

According to an embodiment of the invention, at least one guest dye comprises a chromophore of formula (I), (II), (III), (IV) or (V) salts or stereoisomers thereof 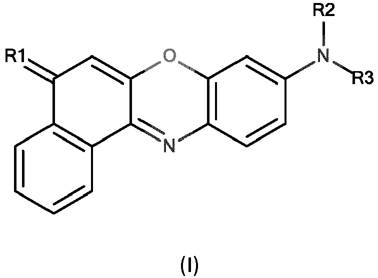 wherein
R1 is selected from the group consisting of =O, =S or =(+)N(R4)(R5), wherein each of R4 and R5 are independently selected from the group consisting of hydrogen, C₁-C₆-alkyl, C₂-C₆-alkenyl and C₂-C₆-alkynyl; and
each of R2 and R3 are independently selected from the group consisting of hydrogen, C₁-C₆-alkyl, C₂-C₆-alkenyl and C₂-C₆-alkynyl; 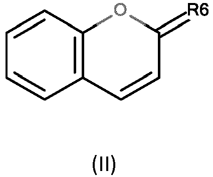
wherein
R6 is selected from the group consisting of =O, =S or =(+)N(R13)(R14), wherein each of R13 and R14 are independently selected from the group consisting of hydrogen, C₁-C₆-alkyl, C₂-C₆-alkenyl and C₂-C₆-alkynyl; 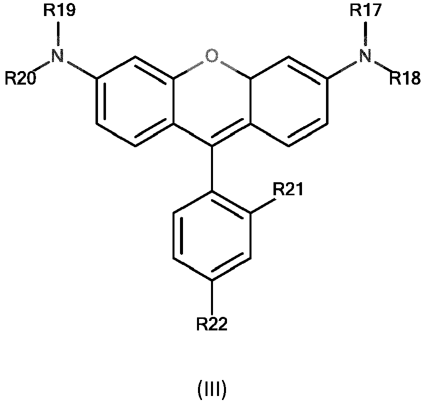 wherein
each of R17, R18, R19 and R20 are independently selected from the group consisting of hydrogen, C₁-C₆-alkyl, C₂-C₆-alkenyl and C₂-C₆-alkynyl; and
each of R21 and R22 is selected from the group consisting of-C(=O)-OH, C(=O)-O-C₁-C₆-alkyl, SO₃H and SO₃N(H)-C1-C12-alkyl; 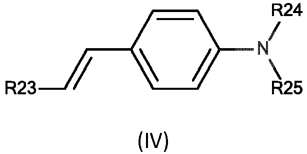
R23 is a C₆-C₃₀ group comprising an aryl group; and
each of R24 and R25 are independently selected from the group consisting of hydrogen, C₁-C₆-alkyl, C₂-C₆-alkenyl and C₂-C₆-alkynyl. 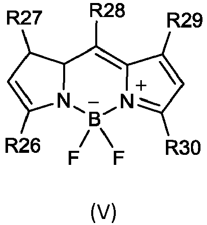
wherein each R26, R27, R28, R29, R30 are independently selected from the group consisting of hydrogen, C₁-C₆-alkyl, optionally substituted with a carbonyl, carboxy or carboxyester, C₂-C₆-alkenyl, C₂-C₆-alkynyl, C₆-C₃₀-aryl, C₇-C₃₀-arylalkyl and C₅-C₁₂-heteroaryl group.

According to this embodiment, the chromophore of formula (I), (II), (III), (IV) or (V) can be attached to the rest of the molecule through any free site

According to a further embodiment of the invention, the at least one guest dye is a compound of formula (VI), stereoisomers or salts thereof 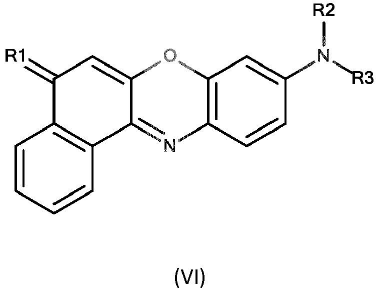 wherein
R1 is selected from the group consisting of =O, =S or =(+)N(R4)(R5), wherein each of R4 and R5 are independently selected from the group consisting of hydrogen, C₁-C₆-alkyl, C₂-C₆-alkenyl and C₂-C₆-alkynyl; and
each of R2 and R3 are independently selected from the group consisting of hydrogen, C₁-C₆-alkyl, C₂-C₆-alkenyl and C₂-C₆-alkynyl.

According to a further embodiment of the invention, the at least one guest dye is a compound of formula (VII), stereoisomers or salts thereof 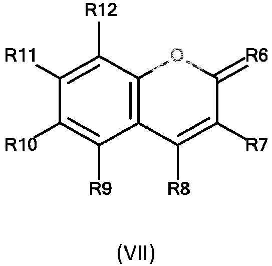 wherein
R6 is selected from the group consisting of =O, =S or =(+)N(R13)(R14), wherein each of R13 and R14 are independently selected from the group consisting of hydrogen, C₁-C₆-alkyl, C₂-C₆-alkenyl and C₂-C₆-alkynyl;
R7 is selected from the group consisting of hydrogen, C₅-C₁₂-aryl, C₅-C₁₂-heteroaryl group, C₁-C₁₂-alkyl, C₂-C₁₂-alkenyl, C₂-C₁₂-alkynyl, C₆-C₁₅-aryl group and C₇-C₃₀-alkylaryl groups, optionally substituted with 1 to 3 groups selected from the group consisting of -OH, C₁-C₆-alkoxy and halogen.
R8 is selected from the group consisting of -OH and C₁-C₆-alkyl optionally perfluorated;
R9 is selected from the group consisting of hydrogen and C₁-C₆-alkyl;
each of R10, R11 and R12 is independently selected from the group consisting of hydrogen, C₁-C₁₂-alkyl, C₂-C₁₂-alkenyl, C₂-C₁₂-alkynyl, C₁-C₁₂-alcoxy and -N(R15)(R16), wherein each of R15 and R16 are independently selected from the group consisting of C₁-C₆-alkyl, C₂-C₆-alkenyl and C₂-C₆-alkynyl; or wherein at least two of R10, R11 and R12 form a cyclic group selected from the group consisting of C₃-C₈ heterocyclyl and C₄-C₈-cycloalkyl.

According to a further embodiment of the invention, the at least one guest dye is a compound of formula (VIII), stereoisomers or salts thereof 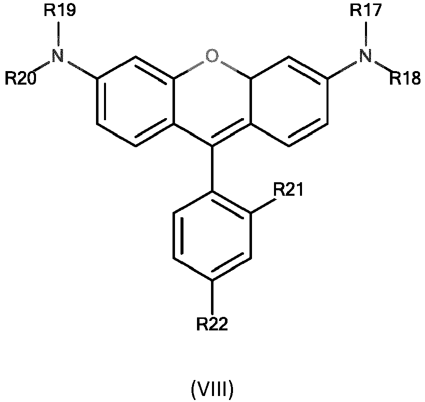 wherein
each of R17, R18, R19 and R20 are independently selected from the group consisting of hydrogen, C₁-C₆-alkyl, C₂-C₆-alkenyl and C₂-C₆-alkynyl; and
each of R21 and R22 is selected from the group consisting of-C(=O)-OH, C(=O)-O-C₁-C₆-alkyl, SO₃H and SO₃N(H)-C1-C12-alkyl.

According to a further embodiment of the invention, the at least one guest dye is a compound of formula (IX), stereoisomers or salts thereof 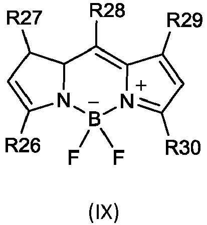 Wherein each R26, R27, R28, R29, R30 are independently selected from the group consisting of hydrogen, C₁-C₆-alkyl, optionally substituted with a carbonyl, carboxy or carboxyester, C₂-C₆-alkenyl, C₂-C₆-alkynyl, C₆-C₃₀-aryl, C₇-C₃₀-arylalkyl and C₅-C₁₂-heteroaryl group.

In all the above examples aromatic groups exist whether or not conjugated to heteroatoms such a nitrogen, oxygen or sulfur. The present invention encompasses all salts of these compounds, which can be represented by different resonant forms placing the charges in different atoms of the molecule. The present invention encompasses all said resonant forms. Said salts can be formed with different inorganic and organic acids, non-limiting examples of which are sulphates; hydrohalide salts; phosphates; lower alkane sulphonates; arylsulphonates; salts of aliphatic mono-, di- or tribasic acids; salts of aromatic acids in which the aromatic nuclei may or may not be substituted with groups such as hydroxyl, alkoxy, amino, mono- or di- lower alkylamino sulphonamido. Also included within the scope of the invention are quaternary salts of the nitrogen atom with lower alkyl halides or sulphates, and oxygenated derivatives of the tertiary nitrogen atom, such as the N-oxides.

According to an embodiment of the invention, R1 is =O or NH₂. According to a further embodiment, R2 and R3 are selected from the group consisting of C₁-C₃-alkyl, preferably ethyl. According to a further preferred embodiment, R1 is =O. According to a further embodiment, each of R17, R18, R19 and R20 are selected from the group consisting of hydrogen and C₁-C₃-alkyl.

Exemplary dyes of the invention are selected from the group consisting of coumarin 30, coumarin 153, coumarin 6, coumarin 343, Rhodamine B, Rhodamine 6G, Rhodamine 123, Nile Red, Nile Blue, 4-(dicyanomethylene)-2-methyl-6-(4-dimethylaminostyryl)-4H-pyran, BODIPYs and mixtures thereof, preferably from the group consisting of coumarin 153, Nile Red, 4-(dicyanomethylene)-2-methyl-6-(4-dimethylaminostyryl)-4H-pyran and mixtures thereof. In a preferred embodiment of the invention the composite material comprises coumarin 153 and Nile Red, or 4-(dicyanomethylene)-2-methyl-6-(4-dimethylaminostyryl)-4H-pyran. Other guest dye which can be used are porphyrins, squarines and mixtures thereof.

BODIPYs are known to the skilled person as those having a boron-dipyrromethene group and can be used in the present invention. Exemplary BODIPYs are those disclosed for example in WO 2014/055505 A1, Si Kyung Yang, Xinghua Shi, Seongjin Park, Taekjip Ha, & Steven C. Zimmerman "A dendritic single-molecule fluorescent probe that is monovalent, photostable and minimally blinking, Nature Chemistry 5, 692-697 (2013), US 8,277,775 B2 or US 6,207,464. Some non-limiting BODIPYs commonly used in the art are {2-[(3,5-Dimethyl-2H-pyrrol-2-ylidene-κN)(4-nitrophenyl)methyl]-3,5-dimethyl-1H-pyrrolato-κN}(difluoro)boron, Difluoro{2-[(3,5-dimethyl-2H-pyrrol-2-ylidene-N)methyl]-3,5-dimethyl-1H-pyrrolato-N}boron, 1,3,5,7-Tetramethyl-8-phenyl-4,4-difluoroboradiazaindacene" 4,4-difluoro-5,7-dimethyl-4-bora-3a,4a-diaza-s-indacene-3-propopionic acid succinimidyl ester, 4,4-difluoro-5,7-diphenyl-4-bora-3a,4a-diaza-s-indacene-3-propopionic acid succinimidyl ester, 4,4-difluoro-5-(2-thienyl)-4-bora-3a,4a-diaza-s-indacene-3-propopionic acid succinimidyl ester, 4,4-difluoro-5-(2-pyrrolyl)-4-bora-3a,4a-diaza-s-indacene-3-propopionic acid succinimidyl ester, 4,4-difluoro-5,7-dimethyl-4-bora-3a,4a-diaza-s-indacene-3-propopionic acid, or 4,4-difluoro-1,3,5,7,8-pentamethyl-4-bora-3a,4a-diaza-s-indacene. Thus, in an embodiment of the invention, R28, R29 and R30 are independently selected from the group consisting of hydrogen and C₁-C₆-alkyl, optionally substituted with a carbonyl, carboxy or carboxyester. According to a further embodiment, R28 is an aryl group, optionally substituted by a nitro group. According to a further embodiment, R26 and R27 are selected from the group consisting of hydrogen, C₁-C₆-alkyl, C₆-C₃₀-aryl, C₇-C₃₀-arylalkyl and C₅-C₁₂-heteroaryl group. According to a further embodiment, R26 is selected from the group consisting of hydrogen, C₁-C₆-alkyl and C₅-C₁₂-heteroaryl; and R27 is selected from the group consisting of hydrogen, C₁-C₆-alkyl and C₆-C₃₀-aryl. According to a further embodiment, R29 is hydrogen of C₁-C₃-alkyl, R30 is a C₁-C₃-alkyl, optionally substituted by carboxyester, preferably forming a succinimidyl ester; R28 is hydrogen, C₁-C₃-alkyl or C₆-C₁₅-aryl, optionally substituted by a nitro group, R27 is hydrogen, C₁-C₃-alkyl or C₆-C₁₅-aryl; and R26 is C₁-C₃-alkyl, C₆-C₁₅-aryl, or C₅-C₁₂-heteroaryl

According to a further embodiment of the invention, said at least one repeat unit is 2,6-naphthalene dicarboxylic acid (i.e. an Zr-NDC MOF) and said at least one guest dye comprises coumarin 153 and Nile Red, or comprises 4-(dicyanomethylene)-2-methyl-6-(4-dimethylaminostyryl)-4H-pyran.

The preparation of the composite materials of the invention comprises two parts: the synthesis of the MOF, previously described in the literature and the inclusion of the dyes into the MOF porous structure. The first part can be done by following standard methods described in the literature (Schaate, A.; Roy, P.; Godt, A.; Lippke, J.; Waltz, F.; Wiebcke, M.; Behrens, P., Modulated Synthesis of Zr-Based Metal-Organic Frameworks: From Nano to Single Crystals. Chemistry - A European Journal 2011, 17 (24), 6643-6651; or Bon, V.; Senkovska, I.; Weiss, M. S.; Kaskel, S., Tailoring of network dimensionality and porosity adjustment in Zr- and Hf-based MOFs. CrystEngComm 2013, 15 (45), 9572-9577), and comprises reacting a salt of the corresponding metal ion, in the present case zirconium, with the organic polydentate bridging ligand (or ligands). This can be done in solution or suspension, or any other form that allows intimate contact between both reactants, and may require sonication and/or heating (see example 1).

Trapping the dyes into the MOF is a simple process wherein the MOF is suspended in a solution of the dye (or dyes) and then washed. If the composite material comprises more than one dye, the process can be repeated with each one, or two or more dyes can be adsorbed simultaneously. Prefered solvents are those capable of disolving the dyes, and include, for example, organic sovents such as ketones, ethers or esters. Typically, the concentration of the at least one guest dye is comprised between 10⁻⁷ M and 10⁻⁴ M. According to a further embodiment, the MOF is present in the solution in a concentration of between 0.1 and 10 grams per ml of solution. The synthetic method is simple and mild. The composite materials obtained display a high quality white light emission under UV-visible irradiation (250-360 nm) giving chromaticity coordinates quite close of the ideal white light one (0.32, 0.34 for Zr-NDC@NR/C153 and 0.32, 0.31 for Zr-NDC@DCM). Therefore, the obtained white light emissive materials can be applied to different fields as light emitting devices or fluorescent sensors.

Further aspects of the invention are a composite material comprising a MOF comprising a plurality of organic polydentate bridging ligands and a plurality of zirconium cations, characterized in that it comprises at least two guest dyes, wherein at least one of said dyes is selected from the group consisting of a compound comprising a naphthoxazinium group, an aromatic cyclobutdione group, a 1-benzopyran-2-one group, xanthenylidene-dialkylammonium group, a porphyrin group or a 4-dimethylaminostyryl group and mixtures thereof. Further aspects include methods of manufacturing said composite material comprising at least two dyes, which are analogous to the second aspect of the invention, as well as their uses as white light emitters, sensors or in imaging, as well as white light emitting devices and sensors.

A further aspect of the invention is a composite material comprising a zirconium and 2,6-naphthalene dicarboxylic acid based MOF (Zr-NDC), characterized in that it comprises at least one guest dye, for example, one, two or three organic guest dyes. Said dyes can be organic laser dyes, or those selected from the group consisting of a compound comprising a naphthoxazinium group, an aromatic cyclobutdione group, a 1-benzopyran-2-one group, xanthenylidene-dialkylammonium group, a porphyrin group or 4-dimethylaminostyryl group or a boron-dipyrrometheneand mixtures thereof. Further aspects include methods of manufacturing said composite material comprising Zr-NDC MOF, which are analogous to the second aspect of the invention, as well as their uses as white light emitters, sensors or in imaging, as well as white light emitting devices and sensors.

The composite materials of the invention can be used to manufacture WLEDs, fluorescent sensors and can be used in imaging. Manufacturing of MOF-based LED can be made in a similar way to that described for other luminescent materials, providing single layer type (Friend, R. H.; Gymer, R. W.; Holmes, A. B.; Burroughes, J. H.; Marks, R. N.; Taliani, C.; Bradley, D. D. C.; Santos, D. A. D.; Bredas, J. L.; Logdlund, M.; Salaneck, W. R., Electroluminescence in conjugated polymers. Nature 1999, 397 (6715), 121-128; and Hung, L. S.; Chen, C. H., Recent progress of molecular organic electroluminescent materials and devices. Materials Science and Engineering: R: Reports 2002, 39 (5-6), 143-222), made of a luminescent layer located between the cathode and anode (usually ITO ones), or the three layer type (Kido, J.; Okamoto, Y., Organo Lanthanide Metal Complexes for Electroluminescent Materials. Chem. Rev. (washington, DC, U. S.) 2002, 102 (6), 2357-2368; and de Bettencourt-Dias, A., Lanthanide-based emitting materials in light-emitting diodes. Dalton Trans. 2007, (22), 2229-2241) which is usually composed by: anode (ITO) / hole transport layer / luminescent layer / electron transport layer / cathode. In these devices, MOF composite could act as the luminescent layer being deposited onto the ITO-covered glass by different methods such as plasma deposition, thermal evaporation, Langmuir-Blodget deposition, or spin casting from solutions. Low work-function metals such as calcium (Ca), magnesium (Mg), or aluminum (Al) and alloys such as Mg: Ag, are commonly used as cathode.

MOF-based luminescent sensors can be based on MOF films which can be fabricated applying different methods as liquid-phase epitaxy, LangmuirBlodgett Layer-by-Layer deposition, electrochemical methods (Betard, A.; Fischer, R., Metalorganic framework thin films: from fundamentals to applications. Chem. Rev. 2012, 112, 1055-1083). The sensing abilities of fluorescent MOFs films towards explosive derivatives have been tested (Lan, A.; Li, K.; Wu, H.; Olson, D. H.; Emge, T. J.; Ki, W.; Hong, M.; Li, J., A Luminescent Microporous Metal-Organic Framework for the Fast and Reversible Detection of High Explosives. Angewandte Chemie International Edition 2009, 48 (13), 2334-2338; and Pramanik, S.; Zheng, C.; Zhang, X.; Emge, T. J.; Li, J., New Microporous Metal-Organic Framework Demonstrating Unique Selectivity for Detection of High Explosives and Aromatic Compounds. J. Am. Chem. Soc. 2011, 133, 4153-4155). These MOFs films exhibit a high luminescence quenching in presence of certain explosive derivatives. In both examples, the material can be fully regenerated by heating at 150 °C. Similar procedure can be followed in the invention described here.

The composite material of the invention can be also used for bioimaging. Similar Zr-based MOF (UiO-66) has been tested interacting with HeLa and J774 cells, showing lower cytotoxicity (Tamames-Tabar, C.; Cunha, D.; Imbuluzqueta, E.; Ragon, F.; Serre, C.; Blanco-Prieto, M. J.; Horcajada, P., Cytotoxicity of nanoscaled metal-organic frameworks. Journal of Materials Chemistry B 2014, 2 (3), 262-271.). Further modifications of the MOF surface by attaching anticancer aptamers (as AS1411) following by the MOF loaded with anticancer drugs can favor their interactions with cancer cells as has been described previously (Deng, K.; Hou, Z.; Li, X.; Li, C.; Zhang, Y.; Deng, X.; Cheng, Z.; Lin, J., Aptamer-Mediated Up-conversion Core/MOF Shell Nanocomposites for Targeted Drug Delivery and Cell Imaging. Scientific Reports 2015, 5, 7851.).

### EXAMPLES

### Materials

The steady-state UV-visible absorption and fluorescence spectra have been recorded using JASCO V-670 and FluoroMax-4 (Jobin-Yvone) spectrophotometers, respectively. Fluorescence quantum yield of Zr-MOF@dyes suspensions were measured using an integrating sphere setup Quanta from Horiba coupled to FluoroMax-4 (Jobin-Yvone) spectrophotometer. All the experiments were performed at 298 K.

For the spectroscopic studies, the solvent (anhydrous), diethyl ether (DE, 99.9%) was from Sigma-Aldrich and used as received. The samples were prepared by adding 1 mg of the composite material to a 10 mL diethyl ether solution. The obtained suspension was placed into a 1cm quartz cuvette and the fluorescence spectra were obtained upon excitation at the MOF absorption region (300-360 nm). The samples were stirred with a magnetic stirring during the measurements to obtain an homogenous suspension.
DCM = 4-(dicyanomethylene)-2-methyl-6-(4-dimethylaminostyryl)-4H-pyran
NR = Nile Red
C153 = Coumarin 153
DE = diethyl ether
NDC = 2,6-naphthalene dicarboxylic acid

### 1) Synthesis of Zr-NDC MOF.

A Zr-NDC MOF was prepared as previously described in the literature (Schaate, A.; Roy, P.; Godt, A.; Lippke, J.; Waltz, F.; Wiebcke, M.; Behrens, P., Modulated Synthesis of Zr-Based Metal-Organic Frameworks: From Nano to Single Crystals. Chemistry-A European Journal 2011, 17 (24), 6643-6651; and Bon, V.; Senkovska, I.; Weiss, M. S.; Kaskel, S., Tailoring of network dimensionality and porosity adjustment in Zr- and Hf-based MOFs. CrystEngComm 2013, 15 (45), 9572-9577)

ZrCl₄ (0.4 g, 1.7 mmol) in DMF (75 mL) was dispersed by ultrasound at 50-60 °C, acetic acid (2.85 mL, 850 mmol) was added. A DMF solution (25 mL) of the linker 2,6-naphthalene dicarboxylic acid (368 mg, 1,7 mmol) was added to the clear solution in an equimolar ratio with regard to ZrCl₄; finally, water (0.125 mL, 0.007 mmol) was added to the solution. The tightly capped flask is sonicated at 60 °C and kept in a bath at 120 °C under static conditions for 24 h. After 24 h, the solutions were cooled to room temperature and the precipitate was isolated by centrifugation. The solid washed with DMF (10 mL). After standing at room temperature for 2 h, the suspension was centrifuged and the solvent was decanted off. The obtained particles were washed with ethanol several times in the same way as described for washing with DMF. Finally, the solid was dried under reduced pressure (80 °C, 3 h).

An analogous process can be used to prepare similar MOF 3 dimensional structures.

### 2) Preparation of Zr-NDC@DCM host-guest complex

To prepare Zr-NDC@DCM host-guest complex, a DCM diethyl ether solution was added to a 10 mg/10 ml Zr-NDC MOF diethyl ether suspension to a final dye concentration of 1.5x10⁻⁵ M. The mixture was sonicated for 15 minutes. After that, it was washed several times with diethyl ether and finally collected in a fresh diethyl ether suspension. The loading of DCM was 80%, calculated using the dye absorption spectra before and after its interaction with the MOF.

### 3) Preparation of Zr-NDC@NR/C153 host-guest complex

The host-guest complex Zr-NDC@NR/C153 was prepared by adding NR in DE solution to a 10 mg/10 ml Zr-NDC DE suspension to a final dye concentration of 5x10⁻⁶ M. The mixture was sonicated for 15 minutes, and after that a C153 DE solution was added to the Zr-NDC@NR DE suspension formed previously to a final C153 concentration of 3x10⁻⁶ M, and sonicating again for 15 min. After that, the mixture was washed several times with DE and collected in a fresh DE suspension.

### 4) Zr-NDC@DCM photoluminescence properties

The absorption spectrum of Zr-NDC@DCM reproduces those of DCM and Zr-NDC, exhibiting a broad absorption band around 450 nm due to the DCM guest, and the characteristic vibrational bands (at ^{∼}350 nm) of the Zr-NDC MOF. The fluorescence spectrum exciting at 335 nm (MOF absorption region) presents a dual emission at the blue and red regions. The emission at the bluest part (^{∼}400 nm) corresponds with the Zr-NDC MOF emission and it is a mirror image (showing vibrational bands) of the absorption one. The emission at the reddest part (^{∼}570 nm, DCM emission) is a broad band whose maximum coincides with that obtained for DCM in a pure DE solution. The relative intensity between the maxima of the observed emission band is 4:1 (Zr-NDC:DCM). The observation of DCM emission exciting at the MOF region (335 nm), indicates the occurrence of an energy transfer from MOF to DCM, providing a white light emitting material. This energy transfer is confirmed by the excitation spectrum observing at the reddest part (where Zr-NDC does not emit) which clearly shows the vibrational Zr-NDC absorption bands. The high quality white light emission is shown by its chromatography coordinates (0.32, 0.31) quite similar to that of the ideal white light (0.33, 0.33). The fluorescence quantum yield obtained upon excitation at 335 nm is 0.15±0.03.

### 5) Zr-NDC@NR/C153 photoluminescence properties

The absorption spectrum of Zr-NDC@NR/C153 reproduces those of NR, C153 and Zr-NDC, exhibiting broad absorption bands around 600 and 410 nm due to NR and C153 guest molecules, respectively; and the characteristics vibrational bands (at ^{∼}350 nm) of the Zr-NDC MOF.

The emission spectrum exciting at the MOF region (335 nm) also shows three well differentiated regions. The first one at the bluest part (^{∼}400 nm) corresponds to the Zr-NDC emission and it is a mirror image of the absorption one, showing the vibrational structures. The second band whose maximum is at the green region (^{∼}500 nm) is the emission of the trapped C153, while the third red shifted band (^{∼}670 nm) is due to the emission of the included NR molecules. The relative intensity between the three emission bands is 1/0.5/0.9 (MOF/C153/NR). The observation of the emission from the trapped C153 and NR molecules when exciting at Zr-NDC region is an indication of an energy transfer process from the MOF to the dyes. Both excitation spectra observing at NR (^{∼}675 nm) and C153 (^{∼}500 nm) regions display the vibrational structures absorption bands (at ^{∼}350 nm), corresponding to that of Zr-NDC MOF. This is the confirmation of the energy transfer process. However, when observing at 675 nm (NR region) no signature of C153 absorption is registered, indicating the energy transfer takes place from the MOF to each dye and there is no evidence of a cascaded energy transfer (Zr-NDC→C153→NR).

Exciting the composite material Zr-NDC@NR/C153 at the UV-Vis (250-350 nm) region, a high quality "cool" white light is emitted. The quality of the Zr-NDC@NR/C153 white light is evaluated by its chromatography coordinates (0.32, 0.34) quite similar to that of the ideal white light (0.33, 0.33). The fluorescence quantum yield obtained upon excitation at 350 nm is 0.33±0.02.

## Claims

1. A white light emitting device comprising composite material emitting white light comprising a metal-organic framework (MOF) comprising a plurality of organic polydentate bridging ligands and a plurality of zirconium cations, **characterized in that** said composite material comprises at least one guest dye capable of absorbing energy in a wavelength range emitted by the MOF when said MOF is excited, said at least one guest dye comprising a chromophore selected from the group consisting of a naphthoxazinium group, an aromatic cyclobutadione group, a 1-benzopyran-2-one group, xanthenylidene-dialkylammonium group, a porphyrin group, a 4-dimethylaminostyryl group, or a boron-dipyrromethene group and mixtures thereof.

2. The white light emitting device according to claim 1, wherein said plurality of organic polydentate bridging ligands comprises a dicarboxylic aromatic compound.

3. The white light emitting device according to any of claims 1 or 2, wherein the at least one guest dye comprises a chromophore of formula (I), (II), (III), (IV) or (V), salts or stereoisomers thereof 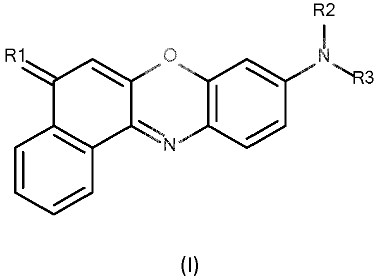 wherein
R1 is selected from the group consisting of =O, =S or =(+)N(R4)(R5), wherein each of R4 and R5 are independently selected from the group consisting of hydrogen, C₁-C₆-alkyl, C₂-C₆-alkenyl and C₂-C₆-alkynyl; and
each of R2 and R3 are independently selected from the group consisting of hydrogen, C₁-C₆-alkyl, C₂-C₆-alkenyl and C₂-C₆-alkynyl; 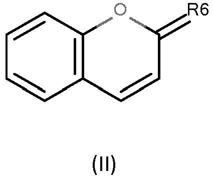
wherein
R6 is selected from the group consisting of =O, =S or =(+)N(R13)(R14), wherein each of R13 and R14 are independently selected from the group consisting of hydrogen, C₁-C₆-alkyl, C₂-C₆-alkenyl and C₂-C₆-alkynyl; 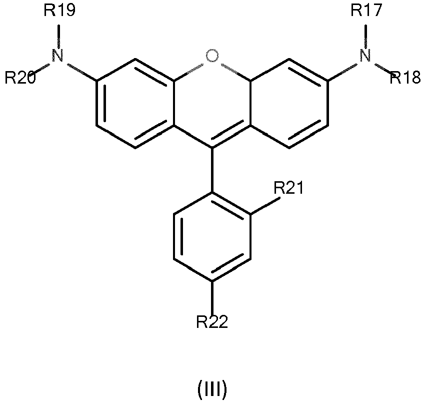 wherein
each of R17, R18, R19 and R20 are independently selected from the group consisting of hydrogen, C₁-C₆-alkyl, C₂-C₆-alkenyl and C₂-C₆-alkynyl; and
each of R21 and R22 is selected from the group consisting of-C(=O)-OH, C(=O)-O-C₁-C₆-alkyl, SO₃H and SO₃N(H)-C1-C12-alkyl; 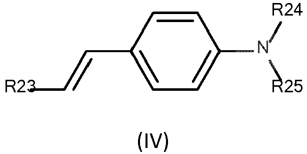
R23 is a C₆-C₃₀ group comprising an aryl group; and
each of R24 and R25 are independently selected from the group consisting of hydrogen, C₁-C₆-alkyl, C₂-C₆-alkenyl and C₂-C₆-alkynyl. 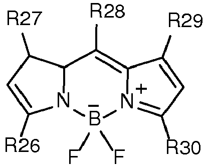
Wherein each R26, R27, R28, R29, R30 are independently selected from the group consisting of hydrogen, C₁-C₆-alkyl, optionally substituted with a carbonyl, carboxy or carboxyester, C₂-C₆-alkenyl, C₂-C₆-alkynyl, C₆-C₃₀-aryl, C₇-C₃₀-arylalkyl and C₅-C₁₂-heteroaryl group.

4. The white light emitting device according to any of claims 1 to 3, wherein the at least one guest dye is a compound of formula (VI), stereoisomers or salts thereof 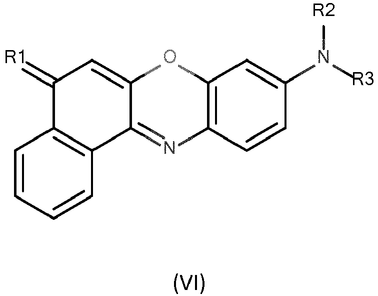 wherein
R1 is selected from the group consisting of =O, =S or =(+)N(R4)(R5), wherein each of R4 and R5 are independently selected from the group consisting of hydrogen, C₁-C₆-alkyl, C₂-C₆-alkenyl and C₂-C₆-alkynyl; and
each of R2 and R3 are independently selected from the group consisting of hydrogen, C₁-C₆-alkyl, C₂-C₆-alkenyl and C₂-C₆-alkynyl;
or
a compound of formula (VII), stereoisomers or salts thereof 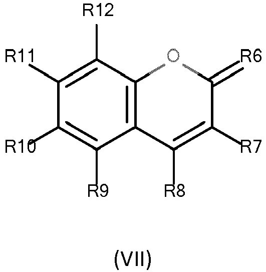 wherein
R6 is selected from the group consisting of =O, =S or =(+)N(R13)(R14), wherein each of R13 and R14 are independently selected from the group consisting of hydrogen, C₁-C₆-alkyl, C₂-C₆-alkenyl and C₂-C₆-alkynyl;
R7 is selected from the group consisting of hydrogen, C₅-C₁₂ aryl, C₅-C₁₂-heteroaryl group, C₁-C₁₂-alkyl, C₂-C₁₂-alkenyl, C₂-C₁₂-alkynyl, C₆-C₁₅-aryl group and C₇-C₃₀-alkylaryl groups, optionally substituted with 1 to 3 groups selected from the group consisting of -OH, C₁-C₆-alkoxy and halogen;
R8 is selected from the group consisting of -OH and C₁-C₆-alkyl optionally perfluorated;
R9 is selected from the group consisting of hydrogen and C₁-C₆-alkyl;
each of R10, R11 and R12 is independently selected from the group consisting of hydrogen, C₁-C₁₂-alkyl, C₂-C₁₂-alkenyl, C₂-C₁₂-alkynyl, C₁-C₁₂-alcoxy and -N(R15)(R16), wherein each of R15 and R16 are independently selected from the group consisting of C₁-C₆-alkyl, C₂-C₆-alkenyl and C₂-C₆-alkynyl; or wherein at least two of R10, R11 and R12 form a cyclic group selected from the group consisting of C₃-C₈ heterocyclyl and C₄-C₈-cycloalkyl;
or
a compound of formula (VIII), stereoisomers or salts thereof 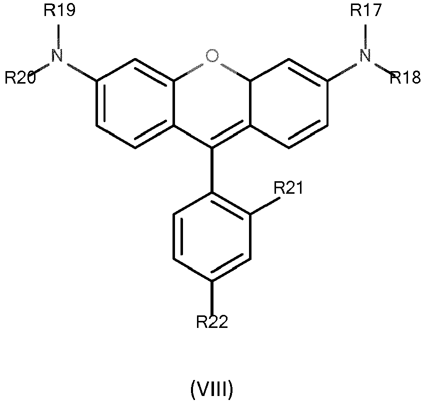 wherein
each of R17, R18, R19 and R20 are independently selected from the group consisting of hydrogen, C₁-C₆-alkyl, C₂-C₆-alkenyl and C₂-C₆-alkynyl; and
each of R21 and R22 is selected from the group consisting of-C(=O)-OH, C(=O)-O-C₁-C₆-alkyl, SO₃H and SO₃N(H)-C1-C12-alkyl.
or 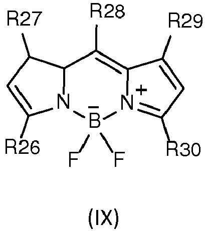
Wherein each R26, R27, R28, R29, R30 are independently selected from the group consisting of hydrogen, C₁-C₆-alkyl, optionally substituted with a carbonyl, carboxy or carboxyester, C₂-C₆-alkenyl, C₂-C₆-alkynyl, C₆-C₃₀-aryl, C₇-C₃₀-arylalkyl and C₅-C₁₂-heteroaryl group.

5. The white light emitting device according to any of claims 1 to 4, wherein said at least one guest dye is selected from the group consisting of coumarin 30, coumarin 153, coumarin 6, coumarin 343, Rhodamine B, Rhodamine 6G, Rhodamine 123, Nile Red, Nile Blue, 4-(dicyanomethylene)-2-methyl-6-(4-dimethylaminostyryl)-4H-pyran, a boron-dipyrromethene group and mixtures thereof.

6. The white light emitting device according to any of claims 1 to 5, wherein said MOF comprises zirconium cation and 2,6-naphthalene dicarboxylic acid, and said at least one guest dye comprises coumarin 153 and Nile Red, or comprises 4-(dicyanomethylene)-2-methyl-6-(4-dimethylaminostyryl)-4H-pyran.

7. The white light emitting device according to any of claims 1 to 6, wherein said energy absorption takes place in at least one single step from the MOF to one of the dyes, takes place in cascade from the MOF to a first dye and from said first dye to a second dye, or wherein said energy absorption takes place in a combination of at least one single steps and at least one cascade.

8. The white light emitting device according to any of claims 1 to 7, wherein the MOF emits a wavelength comprised between 350 and 550 nm.

9. A sensor comprising the white light emitting device as defined in any of claims 1 to 8.

10. Use of the white light emitting device as defined in any of claims 1 to 8 for manufacturing a sensor.

## Patentansprüche

1. Weißlicht emittierende Vorrichtung umfassend Verbundmaterial, das Weißlicht emittiert, umfassend ein metallorganisches Gerüst (MOF) umfassend eine Mehrzahl organischer mehrzähniger Überbrückungsliganden und eine Mehrzahl von Zirkoniumkationen, **dadurch gekennzeichnet, dass** das Verbundmaterial mindestens einen Gastfarbstoff umfasst, der in der Lage ist, Energie in einem Wellenlängenbereich zu absorbieren, der durch das MOF emittiert wird, wenn das MOF angeregt wird, wobei mindestens ein Gastfarbstoff ein Chromophor umfasst ausgewählt aus der Gruppe bestehend aus einer Naphthoxaziniumgruppe, einer aromatischen Cyclobutadiongruppe, einer 1-Benzopyran-2-ongruppe, Xanthenylidendialkylammoniumgruppe, einer Porphyringruppe, einer 4-Dimethylaminostyrylgruppe oder einer Bordipyrromethengruppe und Mischungen davon.

2. Weißlicht emittierende Vorrichtung nach Anspruch 1, wobei die Mehrzahl organischer mehrzähniger Überbrückungsliganden eine aromatische Dicarbonsäureverbindung umfasst.

3. Weißlicht emittierende Vorrichtung nach irgendeinem der Ansprüche 1 oder 2, wobei der mindestens eine Gastfarbstoff ein Chromophor der Formel (I), (II), (III), (IV) oder (V), Salze oder Stereoisomere davon umfasst 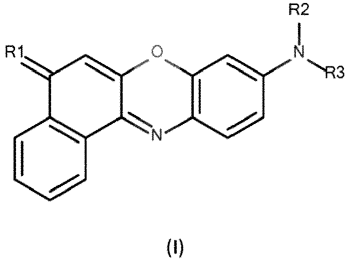 wobei
R1 aus der Gruppe ausgewählt ist bestehend aus =O, =S oder =(+)N(R4) (R5), wobei jedes von
R4 und R5 unabhängig aus der Gruppe ausgewählt ist bestehend aus Wasserstoff, C₁-C₆-Alkyl, C₂-C₆-Alkenyl und C₂-C₆-Alkynyl; und
jedes von R2 und R3 unabhängig aus der Gruppe ausgewählt ist bestehend aus Wasserstoff, C₁-C₆-Alkyl, C₂-C₆-Alkenyl und C₂-C₆-Alkynyl; 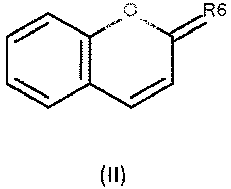
wobei
R6 aus der Gruppe ausgewählt ist bestehend aus =O, =S oder =(+)N(R13)(R14), wobei jedes von R13 und R14 unabhängig aus der Gruppe ausgewählt ist bestehend aus Wasserstoff, C₁-C₆-Alkyl, C₂-C₆-Alkenyl und C₂-C₆-Alkynyl; und 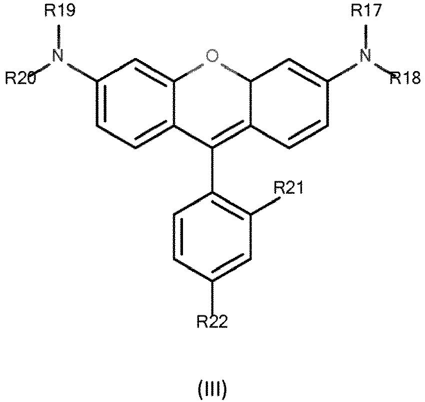
wobei
jedes von R17, R18, R19 und R20 unabhängig aus der Gruppe ausgewählt ist bestehend aus Wasserstoff, C₁-C₆-Alkyl, C₂-C₆-Alkenyl und C₂-C₆-Alkynyl; und
jedes von R21 und R22 aus der Gruppe ausgewählt ist bestehend aus -C(=O)-OH, C(=O)-O-C₁-C₆-Alkyl, SO₃H und SO₃N(H)-C1-C12-Alkyl; 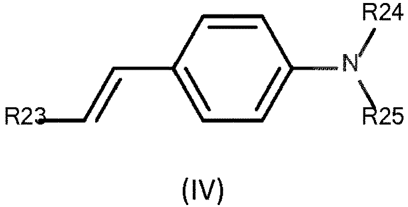
R23 eine C₆-C₃₀-Gruppe ist umfassend eine Arylgruppe; und
jedes von R24 und R25 unabhängig aus der Gruppe ausgewählt ist bestehend aus Wasserstoff, C₁-C₆-Alkyl, C₂-C₆-Alkenyl und C₂-C₆-Alkynyl; 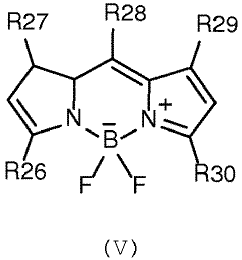
wobei jedes R26, R27, R28, R29, R30 unabhängig aus der Gruppe ausgewählt ist bestehend aus Wasserstoff, C₁-C₆-Alkyl, das optional mit einer Carbonyl-, Carboxy- oder Carboxyester-, C₂-C₆-Alkenyl-, C₂-C₆-Alkynyl-, C₆-C₃₀-Aryl-, C₇-C₃₀-Arylalkyl- und C₅-C₁₂-Heterorylgruppe substituiert ist.

4. Weißlicht emittierende Vorrichtung nach irgendeinem der Ansprüche 1 bis 3, wobei der mindestens eine Gastfarbstoff eine Verbindung der Formel (VI),Stereoisomere oder Salze davon 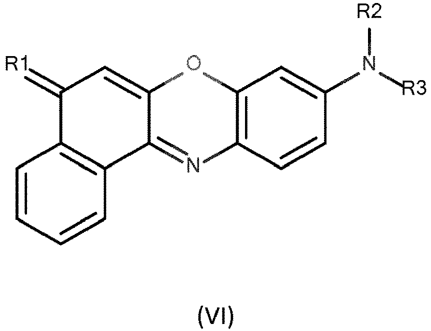 wobei
R1 aus der Gruppe ausgewählt ist bestehend aus =O, =S oder =(+)N(R4)(R5), wobei jedes von
R4 und R5 unabhängig aus der Gruppe ausgewählt sind bestehend aus Wasserstoff, C₁-C₆-Alkyl, C₂-C₆-Alkenyl und C₂-C₆-Alkynyl; und
jedes von R2 und R3 unabhängig aus der Gruppe ausgewählt ist bestehend aus Wasserstoff, C₁-C₆-Alkyl, C₂-C₆-Alkenyl und C₂-C₆-Alkynyl;
oder
eine Verbindung der Formel (VII), Stereoisomere oder Salze davon 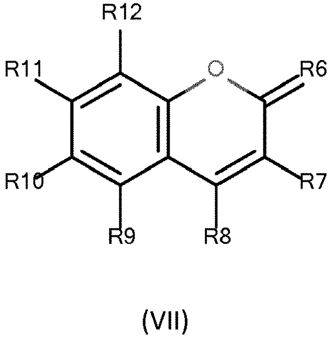
wobei
R6 aus der Gruppe ausgewählt ist bestehend aus =O, =S oder =(+)N(R13)(R14), wobei jedes von R13 und R14 unabhängig aus der Gruppe ausgewählt ist bestehend aus Wasserstoff, C₁-C₆-Alkyl, C₂-C₆-Alkenyl und C₂-C₆-Alkynyl;
R7 aus der Gruppe ausgewählt ist bestehend aus Wasserstoff, einer C₅-C₁₂-Aryl-, C₅-C₁₂-Heteroarylgruppe, C₁-C₁₂-Alkyl-, C₂-C₁₂-Alkenyl-, C₂-C₁₂-Alkynyl-, C₆-C₁₅-Arylgruppe und C₇-C₃₀-Alkylarylgruppen, die optional mit 1 bis 3 Gruppen substituiert sind ausgewählt aus der Gruppe bestehend aus -OH, C₁-C₆-Alkoxy und Halogen;
R8 aus der Gruppe ausgewählt ist bestehend aus -OH und C₁-C₆-Alkyl, das optional perfluoriert ist;
R9 aus der Gruppe ausgewählt ist bestehend aus Wasserstoff und C₁-C₆-Alkyl;
jedes von R10, R11 und R12 unabhängig aus der Gruppe ausgewählt ist bestehend aus Wasserstoff, C₁-C₁₂-Alkyl, C₂-C₁₂-Alkenyl, C₂-C₁₂-Alkynyl, C₁-C₁₂-Alcoxy und-N(R15)(R16), wobei jedes von R15 und R16 unabhängig aus der Gruppe ausgewählt ist bestehend aus C₁-C₆-Alkyl, C₂-C₆-Alkenyl und C₂-C₆-Alkynyl; oder wobei mindestens zwei von R10, R11 und R12 eine cyclische Gruppe bilden ausgewählt aus der Gruppe bestehend aus C₃-C₈-Heterocyclyl und C₄-C₈-Cycloalkyl;
oder
eine Verbindung der Formel (VIII), Stereoisomere oder Salze davon 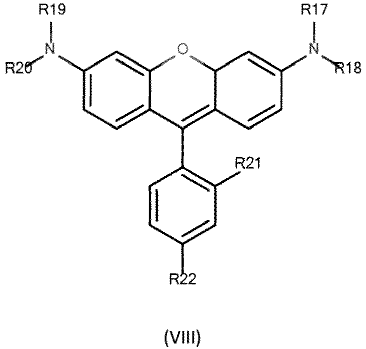
wobei
jedes von R17, R18, R19 und R20 unabhängig aus der Gruppe ausgewählt ist bestehend aus Wasserstoff, C₁-C₆-Alkyl, C₂-C₆-Alkenyl und C₂-C₆-Alkynyl; und
jedes von R21 und R22 aus der Gruppe ausgewählt ist bestehend aus -C(=O)-OH, C(=O)-O-C₁-C₆-Alkyl, SO₃H und SO₃N(H)-C1-C12-Alkyl;
oder 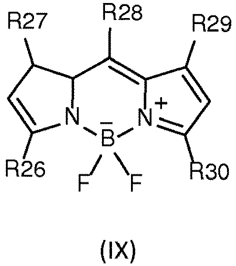
ist, wobei jedes von R26, R27, R28, R29, R30 unabhängig aus der Gruppe ausgewählt ist bestehend aus Wasserstoff, C₁-C₆-Alkyl, das optional mit einer Carbonyl-, Carboxy- oder Carboxyester-, C₂-C₆-Alkenyl-, C₂-C₆-Alkynyl-, C₆-C₃₀-Aryl-, C₇-C₃₀-Arylalkyl- und C₅-C₁₂-Heterorylgruppe substituiert ist.

5. Weißlicht emittierende Vorrichtung nach irgendeinem der Ansprüche 1 bis 4, wobei der mindestens eine Gastfarbstoff aus der Gruppe ausgewählt ist bestehend aus Cumarin 30, Cumarin 153, Cumarin 6, Cumarin 343, Rhodamin B, Rhodamin 6G, Rhodamin 123, Nilrot, Nilblau, 4-(Dicyanomethylen)-2-methyl-6-(4-dimethylaminostyryl)-4H-pyran, einer Bordipyrromethengruppe und Mischungen davon.

6. Weißlicht emittierende Vorrichtung nach irgendeinem der Ansprüche 1 bis 5, wobei das MOF Zirkoniumkation und 2,6-Naphthalindicarbonsäure umfasst und der mindestens eine Gastfarbstoff Cumarin 153 und Nilrot umfasst oder 4-(Dicyanomethylen)-2-methyl-6-(4-dimethylaminostyryl)-4H-pyran umfasst.

7. Weißlicht emittierende Vorrichtung nach irgendeinem der Ansprüche 1 bis 6, wobei die Energieabsorption in mindestens einem einzigen Schritt von dem MOF in einen der Farbstoffe erfolgt, in einer Kaskade von dem MOF zu einem ersten Farbstoff und von dem ersten Farbstoff zu einem zweiten Farbstoff erfolgt oder wobei die Energieabsorption in einer Kombination von mindestens einem einzigen Schritt und mindestens einer Kaskade erfolgt.

8. Weißlicht emittierende Vorrichtung nach irgendeinem der Ansprüche 1 bis 7, wobei das MOF eine Wellenlänge emittiert, die zwischen 350 und 550 nm liegt.

9. Sensor umfassend die Weißlicht emittierende Vorrichtung wie in irgendeinem der Ansprüche 1 bis 8 definiert.

10. Verwendung der Weißlicht emittierenden Vorrichtung wie in irgendeinem der Ansprüche 1 bis 8 definiert, für die Herstellung eines Sensors.

## Revendications

1. Dispositif émettant de la lumière blanche comprenant un matériau composite émettant de la lumière blanche comprenant un réseaux métallo-organiques (MOF) comprenant une pluralité de ligands de pontage polydentés organiques et une pluralité de cations de zirconium, **caractérisé en ce que** le matériau composite comprend au moins un colorant invité capable d'absorber l'énergie dans une gamme de longueurs d'onde émise par le MOF lorsque ledit MOF est excité, ledit au moins un colorant invité comprenant un chromophore choisi dans le groupe consistant en un groupe naphthoxazinium, un groupe aromatique cyclobutadione, un groupe 1-benzopyrane-2-one, un groupe xanthénylidène-dialkylammonium, un groupe porphyrine, un groupe 4-diméthylaminostyryle ou un groupe dipyrrométhène de bore et leurs mélanges.

2. Dispositif émettant de la lumière blanche selon la revendication 1, dans lequel la pluralité de ligands de pontage polydentés organiques comprend un composé aromatique dicarboxylique.

3. Dispositif émettant de la lumière blanche selon l'une quelconque des revendications 1 ou 2, dans lequel le au moins un colorant invité comprend un chromophore de formule (I), (II), (III), (IV) ou (V), des sels ou des stéréoisomères de ceux-ci 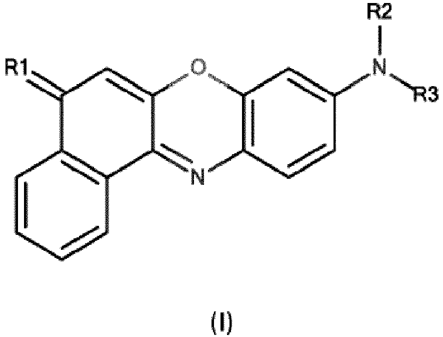 où R1 est choisi dans le groupe consistant en =O, =S ou =(+)N(R4)(R5), où chacun de R4 et R5 est choisi indépendamment dans le groupe consistant en l'hydrogène, un alkyle en C₁-C₆, un alcényle en C₂-C₆ et un alcynyle en C₂-C₆; et
chacun de R2 et R3 est choisi indépendamment dans le groupe consistant en l'hydrogène, un alkyle en C₁-C₆, un alcényle en C₂-C₆ et un alcynyle en C₂-C₆; 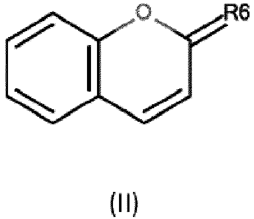 où R6 est choisi dans le groupe consistant en =O, =S ou =(+)N(R13)(R14), où chacun de R13 et R14 est choisi indépendamment dans le groupe consistant en l'hydrogène, un alkyle en C₁-C₆, un alcényle en C₂-C₆ et un alcynyle en C₂-C₆; 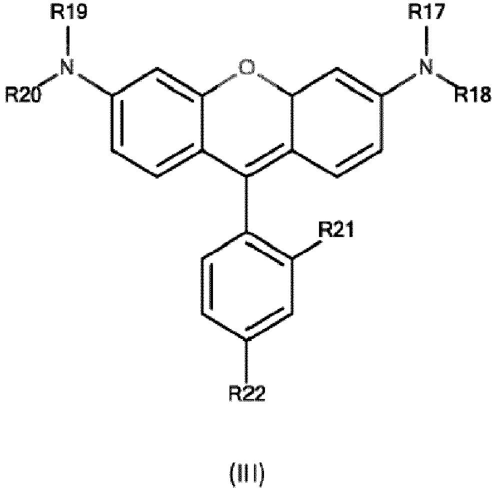 où chacun de R17, R18, R19 et R20 est choisi indépendamment dans le groupe consistant en l'hydrogène, un alkyle en C₁-C₆, un alcényle en C₂-C₆ et un alcynyle en C₂-C₆ ; et
chacun de R21 et R22 est choisi dans le groupe consistant en -C(=0)-OH, C(=0)-0-C₁-C₆-alkyle, SO₃H et SO₃N(H)-C₁-C₁₂-alkyle ; 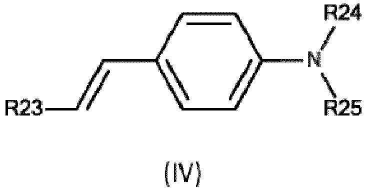 R23 est un groupe en C₆-C₃₀ comprenant un groupe aryle ; et
chacun de R24 et R25 est choisi indépendamment dans le groupe consistant en l'hydrogène, un alkyle en C₁-C₆, un alcényle en C₂-C₆ et un alcynyle en C₂-C₆. 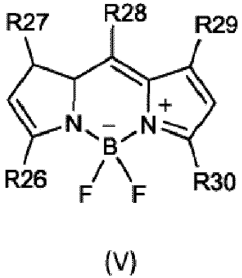 où chacun de R26, R27, R28, R29, R30 est choisi indépendamment dans le groupe consistant en l'hydrogène, un alkyle en C₁-C₆, éventuellement substitué par un carbonyle, un carboxy ou un carboxyester, un alcényle en C₂-C₆, un alcynyle en C₂-C₆, un aryle en C₆-C₃₀, un arylalkyle en C₇-C₃₀ et un groupe hétéroaryle en C₅-C₁₂.

4. Dispositif émettant de la lumière blanche selon l'une quelconque des revendications 1 à 3, dans lequel le au moins un colorant invité est un composé de formule (VI), des stéréoisomères ou des sels de celui-ci 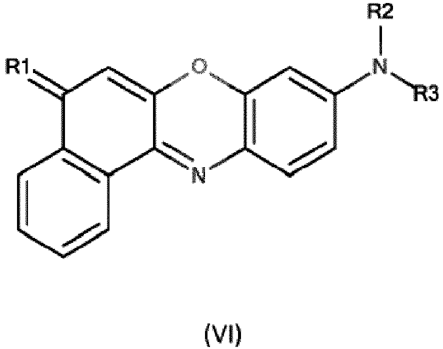 où R1 est choisi dans le groupe consistant en =O, =S ou =(+)N(R4)(R5), où chacun de R4 et R5 est choisi indépendamment dans le groupe consistant en l'hydrogène, un alkyle en C₁-C₆, un alcényle en C₂-C₆ et un alcynyle en C₂-C₆ ; et
chacun de R2 et R3 est choisi indépendamment dans le groupe consistant en l'hydrogène, un alkyle en C₁-C₆, un alcényle en C₂-C₆ et un alcynyle en C₂-C₆ ;
ou
un composé de formule (VII), des stéréoisomères ou des sels de celui-ci 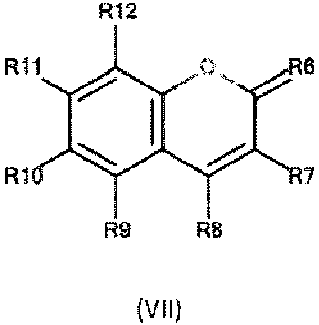 où R6 est choisi dans le groupe consistant en =O, =S ou =(+)N(R13)(R14), dans laquelle chacun de R13 et R14 est choisi indépendamment dans le groupe consistant en l'hydrogène, un alkyle en C₁-C₆, un alcényle en C₂-C₆ et un alcynyle en C₂-C₆ ;
R7 est choisi dans le groupe consistant en l'hydrogène, un aryle en C₅-C₁₂, un groupe hétéroaryle en C₅-C₁₂, un alkyle en C₁-C₁₂, un alcényle en C₂-C₁₂, un alcynyle en C₂-C₁₂, un groupe aryle en C₆-C₁₅ et un groupe alkylaryle en C₇-C₃₀, éventuellement substitués par 1 à 3 groupes choisis dans le groupe consistant en -OH, un alcoxyle en C₁-C₆ et un halogène ;
R8 est choisi dans le groupe consistant en -OH et un alkyle en C₁-C₆ éventuellement perfluoré ;
R9 est choisi dans le groupe consistant en l'hydrogène et un alkyle en C₁-C₆ ; chacun de R10, R11 et R12 est choisi indépendamment dans le groupe consistant en l'hydrogène, un alkyle en C₁-C₁₂, un alcényle en C₂-C₁₂, un alcynyle en C₂-C₁₂, un alcoxyle en C₁-C₁₂ et -N(R15)(R16), où chacun de R15 et R16 est choisi indépendamment dans le groupe consistant en un alkyle en C₁-C₆, un alcényle en C₂-C₆ et un alcynyle en C₂-C₆ ; ou dans lequel au moins deux parmi R10, R11 et R12 forment un groupe cyclique choisi dans le groupe consistant en un hétérocyclyle en C₃-C₈ et un cycloalkyle en C₄-C₈ ;
ou
un composé de formule (VIII), des stéréoisomères ou des sels de celui-ci 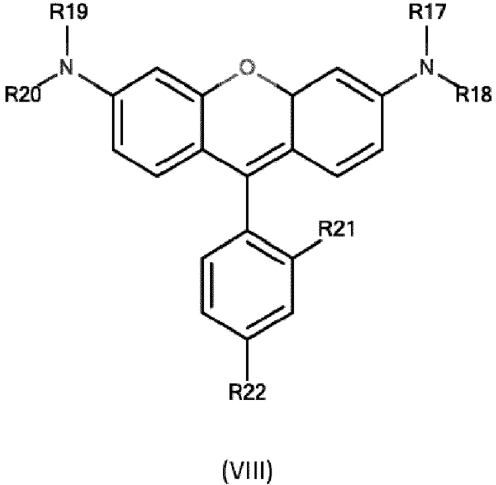 où chacun de R17, R18, R19 et R20 est choisi indépendamment dans le groupe consistant en l'hydrogène, un alkyle en C₁-C₆, un alcényle en C₂-C₆ et un alcynyle en C₂-C₆ ; et
chacun de R21 et R22 est choisi dans le groupe consistant en -C(=0)-OH, C(=0)-O-C₁-C₆-alkyle, SO₃H et SO₃N(H)-C₁-C₁₂-alkyle
ou 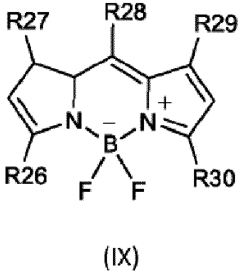 où chacun de R26, R27, R28, R29, R30 est choisi indépendamment dans le groupe consistant en l'hydrogène, un alkyle en C₁-C₆, éventuellement substitué par un carbonyle, un carboxyle ou un carboxyester, un alcényle en C₂-C₆, un alcynyle en C₂-C₆, un aryle en C₆-C₃₀, un arylalkyle en C₇-C₃₀ et groupe hétéroaryle en C₅-C₁₂.

5. Dispositif émettant de la lumière blanche selon l'une quelconque des revendications 1 à 4, dans lequel le au moins un colorant invité est choisi dans le groupe consistant en la coumarine 30, la coumarine 153, la coumarine 6, la coumarine 343, la rhodamine B, la rhodamine 6G, la rhodamine 123, le rouge de Nil, le bleu de Nil, le 4-(dicyanométhylène)-2-méthyl-6-(4-diméthylaminostyryl)-4H-pyrane, un groupe dipyrrométhène de bore et des mélanges de ceux-ci.

6. Dispositif émettant de la lumière blanche selon l'une quelconque des revendications 1 à 5, dans lequel le MOF comprend un cation de zirconium et un acide 2,6-naphtalène dicarboxylique, et le au moins un colorant invité comprend de la coumarine 153 et du rouge de Nil, ou comprend du 4-(dicyanométhylène)-2-méthyl-6-(4-diméthylaminostyryl)-4H-pyrane.

7. Dispositif émettant de la lumière banche selon l'une quelconque des revendications 1 à 6, dans lequel l'absorption d'énergie se déroule en au moins une seule étape du MOF à l'un des colorants, se déroule en cascade du MOF à un premier colorant et dudit premier colorant à un second colorant, ou dans lequel l'absorption d'énergie se déroule en une combinaison d'au moins une seule étape et d'au moins une cascade.

8. Dispositif émettant de la lumière blanche selon l'une quelconque des revendications 1 à 7, dans lequel le MOF émet une longueur d'onde comprise entre 350 et 550 nm.

9. Capteur comprenant un dispositif émettant de la lumière blanche tel que défini dans l'une quelconque des revendications 1 à 8.

10. Utilisation d'un dispositif émettant de la lumière blanche tel que défini dans l'une quelconque des revendications 1 à 8 pour fabriquer un capteur.
